# EUROPEAN PATENT APPLICATION

(11) **EP 1 656 894 A1**
(43) Date of publication of application: **17.05.2006**
(21) Application number: 05256936.5
(22) Date of filing: 10.11.2005
(51) Int. Cl.: A61B 17/32, A61B 17/22, A61B 17/16

(54) **Devices for selectively macerating and sculpting tissue**

(30) Priority: 11.11.2004 US 904456
(71) Applicant: Depuy Mitek, Inc., Norwood, MA 02062 (US)
(72) Inventor: McRury, Ian D., Medway Massachusetts 02053 (US); Sengun, Mehmet Z., Framingham Massachusetts 01702 (US); Ranucci, Kevin J., Warwick Rhode Island 02886 (US); Dunn, Douglas W., Mansfield Massachusetts 02048 (US)
(74) Representative: Fisher, Adrian John

(57) **Abstract**

Various surgical fluid jet cutting instruments for selective bulk removal and precision sculpting of tissue are provided. In an exemplary embodiment, the instrument (10) includes a fluid delivery tube (12) having a nozzle (18) for forming a high pressure fluid jet (22), and an evacuation tube (14) having an evacuation port (20) or jet-receiving opening (20) opposite to and spaced apart from the nozzle (18) for receiving the high pressure fluid jet (22). In use, the fluid jet (22) and/or the evacuation port (20) can be moved relative to one another to allow the device to be selectively used for bulk removal of tissue and for precision sculpting of tissue.

## Description

### FIELD OF THE INVENTION

This application relates to high pressure fluid jets for macerating and sculpting tissue.

### BACKGROUND OF THE INVENTION

High pressure fluid jet systems for cutting and ablating tissue are known in the art. Fluid jet cutters focus pressurized fluid to impact desired tissue and thereby emulsify the tissue. The tissue can then be suctioned or otherwise removed from the surgical site. Many devices utilize a closed-loop system that includes a collection tube positioned a distance apart from the fluid jet nozzle for collecting both the fluid jet and the removed tissue.

While known high pressure fluid jet systems are effective, they are generally limited to use in removing bulk tissue. In particular, the positioning of the fluid delivery tube relative to the collection tube on current high pressure fluid jets only allows the removal of tissue that can be positioned between the two tubes within the path of the fluid jet. The fluid collection tube prevents the user from directing the fluid jet toward tissue that is concave, flat, or even slightly convex. Precision sculpting and erosion of tissue is thus difficult to achieve.

Accordingly, there remains a need in this art for an improved high pressure fluid jet for use in bulk removal as well as precision sculpting of tissue.

### SUMMARY OF THE INVENTION

Various fluid jet cutting instruments are provided for selective bulk removal and precision cutting of tissue. In one exemplary embodiment, the instrument includes a fluid delivery tube having a nozzle formed thereon, preferably at a distal end thereof, for forming a high pressure fluid jet, and an evacuation tube having an evacuation port or fluid-jet receiving port formed thereon, preferably at a distal end thereof, for collecting the high pressure fluid jet from the nozzle on the delivery tube. In use, at least one of the nozzle and the evacuation port can be movable relative to one another to allow the instrument to be selectively used for both bulk removal, whereby the tissue is macerated, and for precision sculpting, whereby the tissue is cut. More particularly, by way of non-limiting example, the evacuation tube, or at least a portion thereof, can be movable to move the evacuation port from a first position, in which the nozzle is substantially axially aligned with the evacuation port, to a second position, in which the nozzle is offset from a central axis of the evacuation port. In the first position, the fluid jet delivered from the nozzle can be used for bulk removal to macerate tissue, as the positioning of the nozzle relative to the evacuation tube requires a shear cutting plane of the fluid jet to extend transversely into the tissue surface being macerated. In this position, the instrument is preferably used to macerate soft tissue, such as plica and fat. In the second position, the fluid jet delivered from the nozzle can be used for precision sculpting to cut fine tissue particles because the positioning of the nozzle relative to the evacuation tube allows the shear cutting plane of the fluid jet to be positioned substantially tangential to the tissue surface. The second position is useful, for example, to sculpt hard tissue, such as bone.

The present invention also provides methods for selective bulk removal and precision sculpting of tissue.

### BRIEF DESCRIPTION OF THE DRAWINGS

FIG. 1A is a side cross-sectional view of one embodiment of a portion of a high pressure fluid jet cutting instrument in accordance with the present invention;
FIG. 1B is a perspective view of the distal portion of the high pressure fluid jet cutting instrument shown in FIG. 1A, showing a fluid jet being delivered from a fluid jet delivery tube to a mid-portion of an evacuation port in an evacuation tube for bulk removal of tissue;
FIG. 1C is a top view of the evacuation tube shown in FIG. 1B, showing the fluid jet being delivered to the evacuation port;
FIG. 1D is a perspective view of the distal portion of the high pressure fluid jet cutting instrument shown in FIG. 1B, showing the fluid jet being delivered from the fluid jet delivery tube to a tip portion of the evacuation port in the evacuation tube for precision sculpting of tissue;
FIG. 1E is a top view of the evacuation tube shown in FIG. 1D, showing the fluid jet being delivered to the evacuation port;
FIG. 1F is a perspective view of the distal portion of the high pressure fluid jet cutting instrument shown in FIG. 1B, showing the fluid jet being delivered from the fluid jet delivery tube to a side of the evacuation port in the evacuation tube for precision sculpting of tissue;
FIG. 1G is a top view of the evacuation tube shown in FIG. 1F, showing the fluid jet being delivered to the evacuation port;
FIG. 2 is a schematic illustration of a fluid jet having a cutting shear plane and a maceration zone;
FIG. 3A is a perspective view of a distal portion of another embodiment of a high pressure fluid jet cutting instrument, showing a fluid jet being delivered from a fluid jet delivery tube to a mid-portion of an evacuation port in an evacuation tube for bulk removal of tissue;
FIG. 3B is a top view of the evacuation tube shown in FIG. 3A, showing the fluid jet being delivered to the evacuation port;
FIG. 3C is a perspective view of the distal portion of the high pressure fluid jet cutting instrument shown in FIG. 3A, showing a fluid jet being delivered from the fluid jet delivery tube to the tip portion of the evacuation port in the evacuation tube for precision sculpting of tissue;
FIG. 3D is a top view of the evacuation tube shown in FIG. 3C, showing the fluid jet being delivered to the evacuation port;
FIG. 4 is a side view of the distal portion of the high pressure fluid jet cutting instrument shown in FIG. 1A, showing the fluid jet positioned for precision sculpting of tissue.

### DETAILED DESCRIPTION OF THE INVENTION

Various fluid jet cutting instrument for selective bulk removal and precision sculpting of tissue are provided. In general, an exemplary instrument includes a fluid delivery tube having a nozzle for forming a high pressure fluid jet, and an evacuation tube having an evacuation port or jet-receiving opening opposite to and spaced apart from the nozzle for receiving the high pressure fluid jet. In use, the evacuation port and/or the nozzle can be moved relative to one another to allow the device to be selectively used for bulk removal of tissue to macerate the tissue and for precision sculpting of tissue to cut the tissue. A person skilled in the art will appreciate that the fluid delivery tube and the evacuation tube can have a variety of other configurations, and they can be incorporated into and/or include features present in various other fluid jet cutting instruments known in the art.

The term "bulk removal" and variations thereof is intended to encompass the mass ablation of large quantities of redundant tissue such as, but not limited to fat, fat pad, plica, osteoarthritic tissue, and the term "precision sculpting" and variations thereof is intended to encompass the removal or shaping of functional anatomy which has been damaged or diseased in order to approximate the original shape and functionality. The term "macerate" and variations thereof is intended to encompass crushing between the fluid jet and a portion of the collection tube such that the tissue is ablated (almost formed into a liquefied material), and the term "cut" and variations thereof is intended to encompass removing tissue from the body using the fluid jet such that the tissue is pushed by the jet or entrained within the jet and collected in the collection tube.

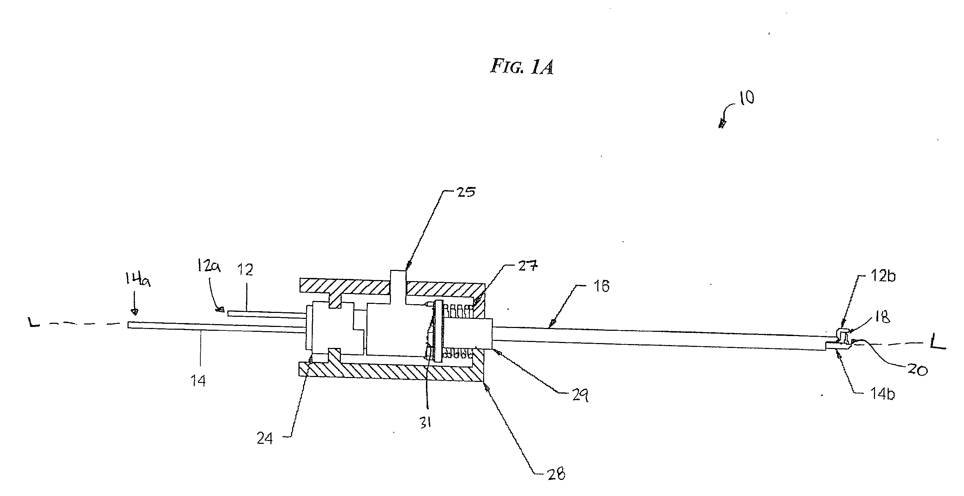

FIG. 1A illustrates one exemplary embodiment of a surgical fluid jet cutting instrument. As shown, the instrument 10 generally includes a fluid delivery tube 12 and an evacuation tube 14. Each tube 12, 14 has a substantially elongated shape with an inner lumen extending therethrough between proximal and distal ends 12a, 12b, 14a, 14b thereof. The tubes 12, 14 can be directly mated to one another, or a portion of each tube 12, 14 can be disposed within an outer housing 16 for retaining the tubes 12, 14 relative to one another, as shown. The outer housing 16 can have virtually any shape and size, and it can optionally be in the form of a handle to facilitate grasping of the device. In the illustrated embodiment, the outer housing 16 functions to allow movement of the fluid delivery tube 12 relative to the evacuation tube 14, as will be discussed in more detail below. The outer housing 16, and in particular the fluid delivery tube 12, is also designed to couple to a high pressure liquid source, such as a high pressure pump or liquid dispenser, for delivering fluid to the fluid delivery tube 12. The outer housing 16, and more particularly the evacuation tube 14, can also optionally be configured to couple to a source of suction, such as a vacuum pump, aspirator, or to a waste canister for collecting fluid and tissue evacuated through the evacuation tube 14. For example, the proximal end 12a, 14a of the fluid delivery tube 12 and the evacuation tube 14 can extend into a handle or another housing that is connected to a fluid delivery source and a suction device.

The distal end 12b, 14b of each tube 12, 14, which is shown in more detail in FIGS. 1B-1G, can also have a variety of configurations. In an exemplary embodiment, the distal end 12b of the fluid delivery tube 12 can be curved away from the evacuation tube 14 such that the fluid delivery tube 12 and the evacuation tube 14 are spaced a distance apart from one another. As further shown, the fluid delivery tube 12 preferably includes a nozzle 18 formed thereon and opposed to the evacuation tube 14 for forming and delivering a high pressure fluid jet 22. The nozzle 18 is in communication with the inner lumen (not shown) such that when the proximal end 12a of the fluid delivery tube 12 is coupled to a high pressure fluid source, fluid can be delivered through the fluid delivery tube 12 to the nozzle 18, which forms a fluid jet 22 having a specific shape and size. The evacuation tube 14 can include an evacuation port 20 for receiving the fluid jet 22, and any tissue contained therein. The evacuation port 20 can extend into the inner lumen extending through the evacuation tube 14 to allow the fluid jet 22 and the tissue to be collected.

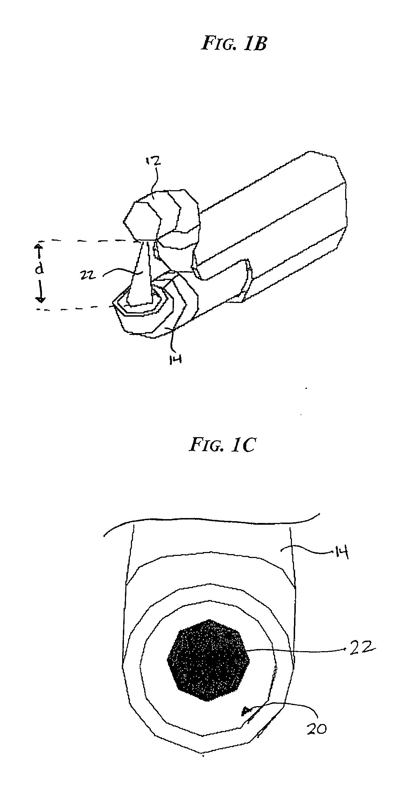

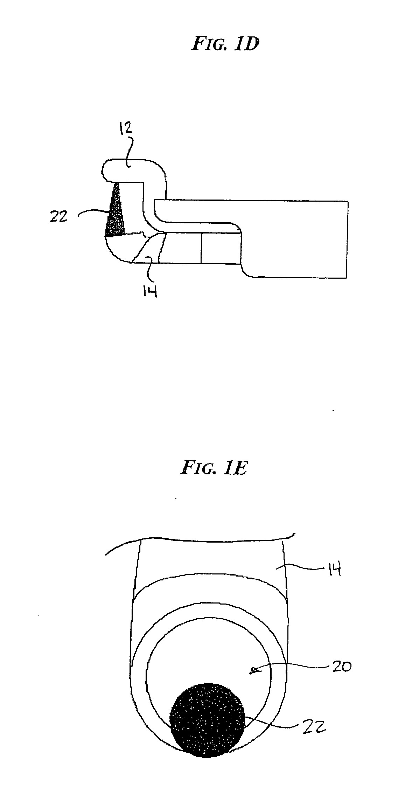

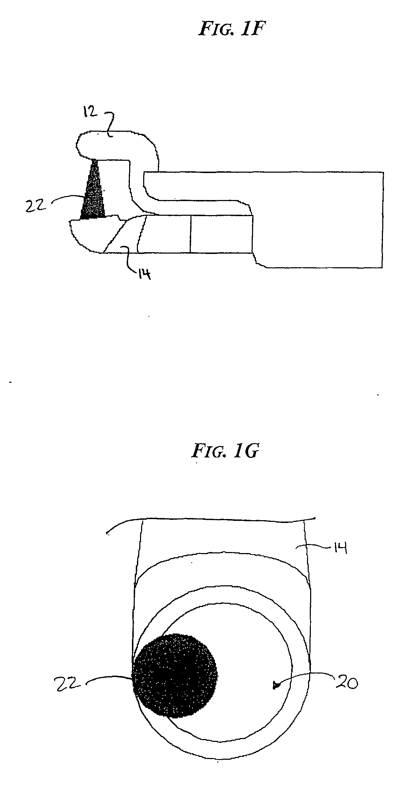

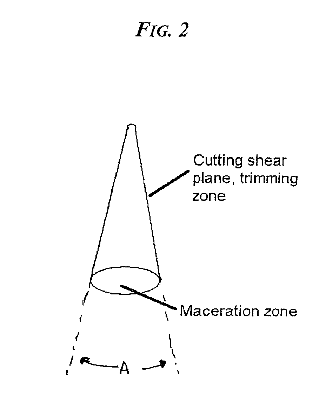

Still referring to FIGS. 1A-1G, the nozzle 18 is preferably positioned a distance apart from and opposite to the evacuation port 20. The distance *d* will depend on the size of the fluid jet, but preferably the distance *d* is configured such that the diameter of the fluid jet 22 as it enters the evacuation port 20 occupies a predetermined area of the evacuation port. While this predetermined area can vary depending on the intended use, in an exemplary embodiment the fluid jet 22 occupies approximately 50% to 60% of the evacuation port 20. The fluid jet 22 can occupy less than 50% of the evacuation port 20, however such a configuration may result in a fluid jet that is effective to sculpt the tissue without being effective to macerate the tissue. Conversely, the fluid jet 22 can occupy more than 60% and up to 100% of the evacuation port 20, however such a configuration may result in a fluid jet that is effective to macerate tissue without being effective to sculpt the tissue. As noted above, while the distanced *d* will vary depending on the size of the fluid jet 22 as well as the desired area of the evacuation port 20 to be occupied by the fluid jet 22, in an exemplary embodiment the distance *d* between the nozzle 18 and the evacuation port 20 is configured such that the fluid jet 22 has a cone angle *A*, shown in FIG. 2, that is in the range of about 15° to 20°, and more preferably that is about 17° to 19°. By way of non-limiting example, the distance *d* can be in the range of about 1 mm to 5 mm. In use, the size of the fluid jet 22 relative to the size of the evacuation port 20 allows the fluid jet 22 to be collected at various locations within the evacuation port 20, thus allowing the instrument 10 to be used to selectively macerate and sculpt tissue. The pressure of the fluid jet 22 can also vary, but in an exemplary embodiment the fluid jet 22 is delivered at a pressure that is in the range of about 1000 PSI to 20,000 PSI, more preferably 5000 PSI to 15,000 PSI.

The evacuation port 20 formed in the evacuation tube 12 can have a variety of shapes and sizes. In the embodiment shown in FIGS. 1A-1G, the evacuation port 20 is substantially circular in shape. In use, the fluid jet 22 can be adjusted both radially and axially with respect to the evacuation port 20. In particular, FIG. 1B illustrates the nozzle 18 on the fluid delivery tube 12 aligned with a mid-portion of the evacuation port 20 such that the fluid jet 22 is collected at the mid-portion of the evacuation port 20, as shown in FIG. 1C. In this position, the fluid jet 22 is particularly suitable for use in bulk removal of tissue, as will be discussed in more detail below. The fluid jet 22 can then be adjusted for use in precision sculpting, as will also be discussed in more detail below. In particular, the fluid delivery tube 12 or the evacuation tube 14 can be axially translated from the position shown in FIG. 1B to the position shown in FIG. 1D. In this position, the fluid jet 22 is delivered to the tip of the evacuation port 20, as shown in FIG. 1E. Alternatively, the fluid delivery tube 12 or the evacuation tube 14 can be radially translated from the position shown in FIG. 1B to the position shown in FIG. 1E wherein the fluid jet 22 is delivered to a side of the evacuation port 20, as shown in FIG. 1F.

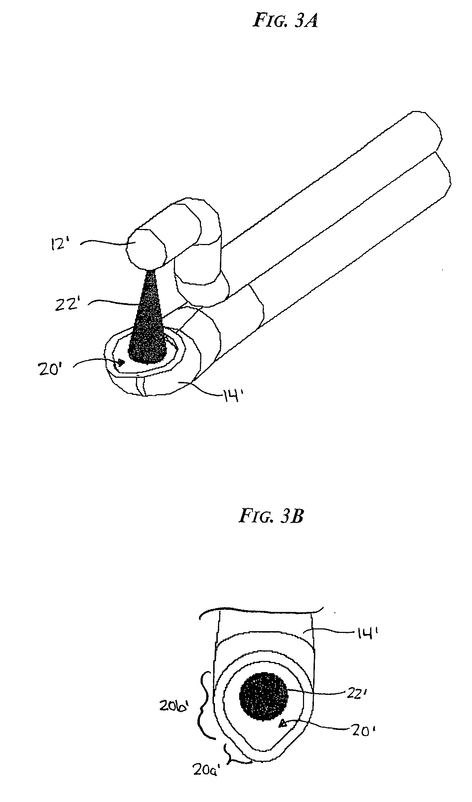

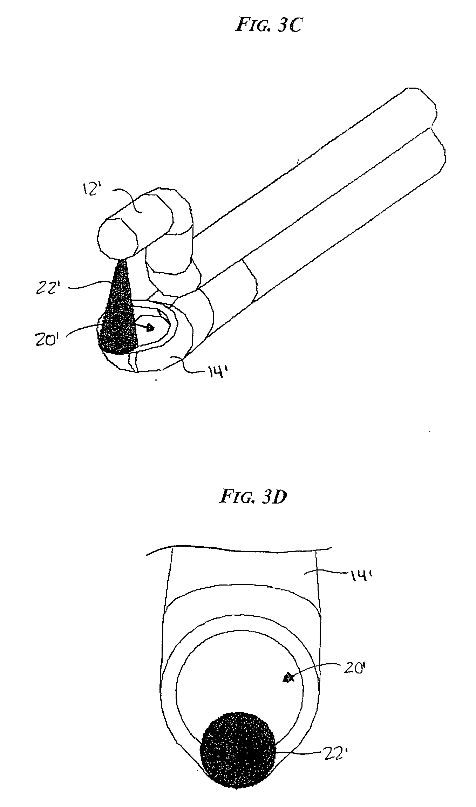

In another embodiment, shown in FIGS. 3A-3D, the evacuation port 20' can be substantially pear-shaped or tear-shaped. In particular, the evacuation port 20' includes a central substantially circular region 20a' and an offset pointed region 20b'. The evacuation tube also preferably has a pear-shape or tear-shape to contour the shape of the evacuation port 20'. In use, the shape allows the fluid jet 22' to be received at various locations in the evacuation port 20'. In particular, FIG. 3A illustrates the fluid delivery tube 12' aligned with a mid-portion of the evacuation port 20' such that the fluid jet 22' is collected at the mid-portion of the evacuation port 20', as shown in FIG. 3B. In this position, the fluid jet 22' is particularly suitable for use in bulk removal of tissue, as will be discussed in more detail below. The fluid jet 22' can then be adjusted for use in precision sculpting, as will also be discussed in more detail below. In particular, the fluid delivery tube 12' or the evacuation tube 14' can be axially translated from the position shown in FIG. 3A to the position shown in FIG. 3C. In this position, the fluid jet 22' is delivered to the tip or pointed region 20b' of the evacuation port 20', as shown in FIG. 3D. While not particularly necessary, the fluid jet 22' can also be moved radially to deliver the fluid jet 22' to a side of the evacuation port 20'.

A variety of techniques can be used to allow movement of the fluid delivery tube 12 relative to the evacuation tube 14. For example, referring back to FIG. 1A, the instrument 10 can include a handle 28 that is coupled to the housing 16 and that receives a portion of the evacuation tube 14 and the fluid delivery tube 12. A cam mechanism is disposed within the handle 28 and it is effective to axially move the fluid delivery tube 12 with respect to the evacuation tube 14. In particular, the cam mechanism includes a radial switch 25 that is rotatably disposed within the handle 28 and that includes a cam ramp 31 formed on a distal end thereof. The cam ramp 31 is coupled to a cam follower 29, which is rigidly attached to the housing 16 and the fluid delivery tube 12, and which is preloaded onto the switch 25 via a spring 27 that is retained inside the handle 28. As a result, when the switch 25 is rotated, the cam ramp 31 forces the cam follower 29 to move axially, thereby axially moving the housing 16 and the fluid delivery tube 12 to adjust the position of the fluid jet 22 with respect to the evacuation port 20 in the evacuation tube 14. The radial switch 25 is also preferably coupled to an evacuation housing 24 that is disposed within the handle 28. This ensures that the housing 16 and the fluid delivery tube 12 move a distance axially that is dictated by the cam ramp 21 on the switch 25.

A person skilled in the art will appreciate that a variety of techniques can be used to effect axial movement of the fluid delivery tube 12 and/or the evacuation tube 14 relative to one another. Moreover, techniques known in the art can also be used to cause the fluid delivery tube 12 and/or the evacuation tube 14 to pivot, rotate, or otherwise move about the longitudinal axis *L* thereof.

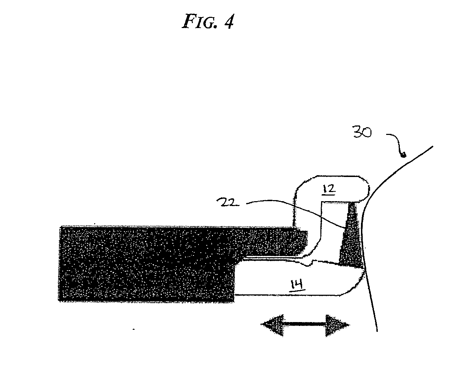

In use, the position of the evacuation port 20 relative to the nozzle 18 can be used to control the effect of the fluid jet 22 on the tissue. First, referring back to FIG. 2, fluid 22 jet is shown in more detail, and as shown the fluid jet 22 includes a shear cutting plane which is formed around a perimeter thereof along a length thereof, and a maceration zone, which is internal to the cutting plane. Thus, when the fluid jet 22 is positioned such that the shear cutting plane is transverse to the tissue surface, i.e., it extends into the tissue surface, the fluid jet 22 can be used for bulk removal of tissue such that the tissue within the maceration zone will be macerated. This is preferably achieved by positioning the fluid jet 22, 22' such that it is collected at a mid-portion of the evacuation port 20, 20', as shown in FIGS. 1B-1C and 3A-3B. Conversely, when the fluid jet 22 is positioned such that the shear cutting plane is substantially tangential to the tissue surface, as shown for example in FIG. 4, the fluid jet 22 can be used for precision sculpting of tissue. Precision sculpting of the tissue can be achieved when the fluid jet 22 is positioned to be collected adjacent to a perimeter of the evacuation port 20, as shown in FIGS. 1D-1G and 3C-3D, such that the fluid delivery tube 12 and the evacuation tube 14 do not interfere with the tangential contact between the fluid jet 22 and the tissue surface 30. Accordingly, by providing a movable fluid delivery tube 12 and/or movable evacuation tube 14, the fluid jet 22 can be selectively positioned for use in bulk removal of tissue and for use in precision sculpting of tissue.

In an exemplary method of using the instrument of the present invention, the fluid jet 22 is first positioned as shown in FIGS. 1B-1C and 3A-3B such that the fluid jet 22 is collected at a substantial mid-portion of the evacuation port. The instrument is then positioned adjacent to a tissue surface such that the high pressure fluid jet 22 extends transversely into the tissue surface for bulk removal of the tissue. The macerated tissue can be collected with the fluid jet 22 in the evacuation port 20 and through the evacuation tube 14. Once the desired amount of tissue is removed, the fluid jet 22 is moved to a second position as shown in FIGS. 1D-1G and 3C-3D such that the fluid jet 22 is collected adjacent to a perimeter of the evacuation port 20. The instrument is then positioned adjacent to a tissue surface 30, as is also shown in FIG. 4, such that the high pressure fluid jet 22 is tangential to the tissue surface 30 for precision sculpting of the tissue. As noted above, the fluid jet 22 and the removed tissue can be collected in the evacuation tube 14.

One skilled in the art will appreciate further features and advantages of the invention based on the above-described embodiments. Accordingly, the invention is not to be limited by what has been particularly shown and described, except as indicated by the appended claims. All publications and references cited herein are expressly incorporated herein by reference in their entirety.

## Claims

1. A fluid jet cutting instrument, comprising:
a fluid delivery tube having a nozzle for forming a fluid jet and an evacuation tube coupled to the fluid delivery tube and having an evacuation port positioned opposite to and spaced apart from the nozzle for collecting the fluid jet from the nozzle, at least one of the fluid delivery tube and the evacuation tube being movable relative to one another to position the nozzle in a first position, in which a fluid jet formed by the nozzle is received at a substantial mid-portion of the evacuation port, and a second position, in which a fluid jet formed by the nozzle is received at a location that is offset from the substantial mid-portion of the evacuation port.

2. The instrument of claim 1, wherein a fluid jet formed by the nozzle is adapted to be positioned substantially tangential to a target tissue surface when the nozzle is in the second position.

3. The instrument of claim 1, wherein at least one of the fluid delivery tube and the evacuation tube are slidably movable along a longitudinal axis thereof relative to the evacuation tube.

4. The instrument of claim 1, wherein at least a portion of at least one of the fluid delivery tube and the evacuation tube are radially movable along a longitudinal axis thereof.

5. The instrument of claim 1, further comprising a housing disposed around a portion of the fluid delivery tube and the evacuation tube, and a rotatable mechanism coupled to the housing and effective to axially move at least one of the fluid delivery tube and the evacuation tube relative to one another.

6. The instrument of claim 1, wherein the evacuation port in the evacuation tube comprises a substantially circular opening extending into a lumen formed through the evacuation tube.

7. The instrument of claim 1, wherein the evacuation port in the evacuation tube is substantially pear-shaped.

8. The instrument of claim 1, wherein a fluid jet formed by the nozzle is received adjacent to a perimeter of the evacuation port when the nozzle is positioned in the second position.

9. A surgical fluid jet cutting instrument, comprising:
a fluid delivery tube having a nozzle for forming a fluid jet; and
an evacuation tube having a jet-receiving opening opposite to the nozzle for receiving a fluid jet formed by the nozzle, the evacuation tube and the fluid delivery tube being movably coupled to one another such that the nozzle can be moved between a first position in which a fluid jet formed by the nozzle is adapted for bulk removal of tissue, and a second position in which a fluid jet formed by the nozzle is adapted for precision sculpting of tissue.

10. The instrument of claim 9, wherein, when the nozzle is positioned in the second position the fluid jet formed by the nozzle is adapted to be positioned tangential to a tissue surface for precision sculpting of the tissue.

11. The instrument of claim 9, wherein the fluid delivery tube is slidably movable along a longitudinal axis thereof relative to the evacuation tube.

12. The instrument of claim 11, wherein at least a portion of the fluid delivery tube is pivotally movable along a longitudinal axis thereof relative to the evacuation tube.

13. The instrument of claim 9, wherein the jet-receiving opening in the evacuation tube is substantially pear-shaped.

14. The instrument of claim 13, wherein the substantially pear-shaped jet-receiving opening includes a central substantially circular region and an offset pointed region, and wherein the fluid delivery tube is movable between a first position, in which a fluid jet formed by the nozzle is directed into the central substantially circular region, and a second position, in which a fluid jet formed by the nozzle is directed into the offset pointed region.
